Europäisches Patentamt

European Patent Office

Office européen des brevets

(19)

(11) Publication number: **0 164 743**
A2

# (12) EUROPEAN PATENT APPLICATION

(21) Application number: 85107247.0

(22) Date of filing: 12.06.85

(51) Int. Cl.⁴: **A 61 F 13/18**

(30) Priority: 15.06.84 US 620935

(43) Date of publication of application:
18.12.85 Bulletin 85/51

(84) Designated Contracting States:
BE DE FR GB IT NL

(71) Applicant: GRAIN PROCESSING CORPORATION
1600 Oregon Street
Muscatine Iowa 52761(US)

(72) Inventor: Brogly, Donald A.
2813 Highland Court
Muscatine Iowa 52761(US)

(74) Representative: Klunker, Hans-Friedrich, Dr. et al,
Patentanwälte Klunker, Schmitt-Nilson, Hirsch
Winzererstrasse 106
D-8000 München 40(DE)

(54) Absorbent laminates.

(57) Laminated absorbent structures having an absorbent material confined therein are produced by distributing over the surface of a wetted substrate tissue an absorbent material in granular or powdered form. A top or overlay ply of tissue is then applied over the absorbent and the two plies of lamina are then pressed together and heated to form a bonded laminate with the absorbent serving to maintain a good bond between the substrate and overlay lamina.

EP 0 164 743 A2

./...

- 1 -

## ABSORBENT LAMINATES

The present invention relates to the production of a laminate of two or more layers of tissue-like material in such manner that fixed between the lamina is a layer of liquid absorbing agent in granular form. The liquid absorbing agent is distributed between the layers and fixed in position so that migration of the agent between the layers is inhibited but, nevertheless, retains its ability to absorb liquid. One or more of the tissue-like layers used in the laminate have certain desired characteristics with respect to wet strength, surface texture and hydrophobicity.

In the last few years certain hydrocolloidal absorbents have been developed which have the ability to absorb large quantities of liquids including, e.g., body fluids. The use of these absorbents in disposable softgoods products such as diapers, dressings, sanitary napkins, catamenial tampons and the like offers advantages. When used in disposable softgoods products, these materials result in better absorbency thereby permitting reduction in the bulk of the disposable product by allowing the manufacturer to decrease the amount of cellulose wadding or fluff pulp in the disposable product. Also, since they generally possess greater affinity for liquid than does cellulosic material, these polymer materials retain the fluid better in a disposable product under pressure. Thus, disposable softgoods products containing the hydrocolloidal absorbents show better performance when pressure is applied to them than is the case with the cellulosic product. This is exemplified when a patient lies on an underpad or a child sits or otherwise places pressure on a disposable diaper.

The cost of these absorbents relative to other less efficient absorbents, i.e., cellulose, dictates their incorporation into liquid-absorbing articles in a controlled manner in order to achieve cost effective performance. This has led to the development of a number of web form absorbent laminates such as disclosed in United States patent No. 4,117,184 (film-tissue laminate) and in United States patent No. 4,260,443 (powder-tissue laminate).

Such laminates are usually formed by moistening the absorbent film or post wetting the laminate structure. Film laminates are formed at relatively low line speeds, such as 80-100 feet per minute, due to high amounts of water inherent in the casting operation and require additional treatments such as, for example, crushing as disclosed in United States patent No. 4,293,609, to make them satisfactory for many applications. Post wetting of an embossed laminate structure using a spot application of water results in a product which tends to delaminate on further converting operations (i.e., rewinding and slitting). The resultant product also retains the undesirable properties of rattle and coarse texture.

There are problems associated with the use of the hydrocolloid polymers in disposable sanitary products. For example, when the hydrocolloidal polymers, especially in powder form, are added to disposable softgoods, the polymers are dusty during application and shift in the finished product. The shifting of the hydrocolloidal particles takes place during the manufacture, handling, storage and use of the article. This can result in the particles bunching together and failing to be evenly distributed throughout the article. United States patent No. 4,260,443 addresses these problems by having the lamina secured together at spaced points with the majority of the absorbent particles located in pockets between such adhering points.  Even here, however, the absorbent can accumulate in the pockets and not provide uniform distribution throughout the laminate.

It is a primary object of this invention to provide an improved process for producing laminated structures containing liquid-absorbing absorbents.

A further object of the invention is to provide a process for producing high-capacity liquid-absorbing laminated structures which can be produced at high speed using readily available equipment.

A still further object of the invention is to provide high-capacity liquid-absorbing laminated structures which exhibit good bond between the lamina and which laminates exhibit desirable attributes with respect to hand feel and drape.

A still further object of the invention is to provide high-capacity liquid-absorbing laminated structures wherein the lamina forming the structure are bonded together over substantially the entire surface thereof and a liquid absorbent is evenly distributed throughout the laminate.

In accordance with this invention laminated absorbent structures having an absorbent material confined therein are produced at high line speeds, such as on the order of 61-122 meters per minute, in a very simple manner using conventional equipment for forming laminated tissue products.

The improved process of this invention is carried out by distributing a metered amount of an absorbent material in granular or powdered form over the surface of a substrate tissue having particular characteristics which has been maintained or wetted with water prior to application of the absorbent.  The substrate tissue employed is one having high wet strength to permit wetting prior to application of the absorbent and to resist tearing during production at high line speeds.  Moreover, it should have the ability to retain, prior to addition of the absorbent, an amount of water which is sufficient to achieve a good bond strength in the laminate but which does not adversely affect the absorbance of the finished laminate.

A top or overlay ply of tissue is then applied over the absorbent and the two plies of lamina are then pressed together and heated to form a bonded laminate with the absorbent serving to maintain a good bond between the substrate and overlay lamina. The granular absorbent remains evenly distributed throughout the laminate and is readily available to provide absorbability of liquids when the structure is used as a diaper or similar liquid-absorbing article. Upon contact with the completely wetted substrate, the absorbent becomes fixed with respect thereto with little tendency to migrate or bunch during subsequent processing.

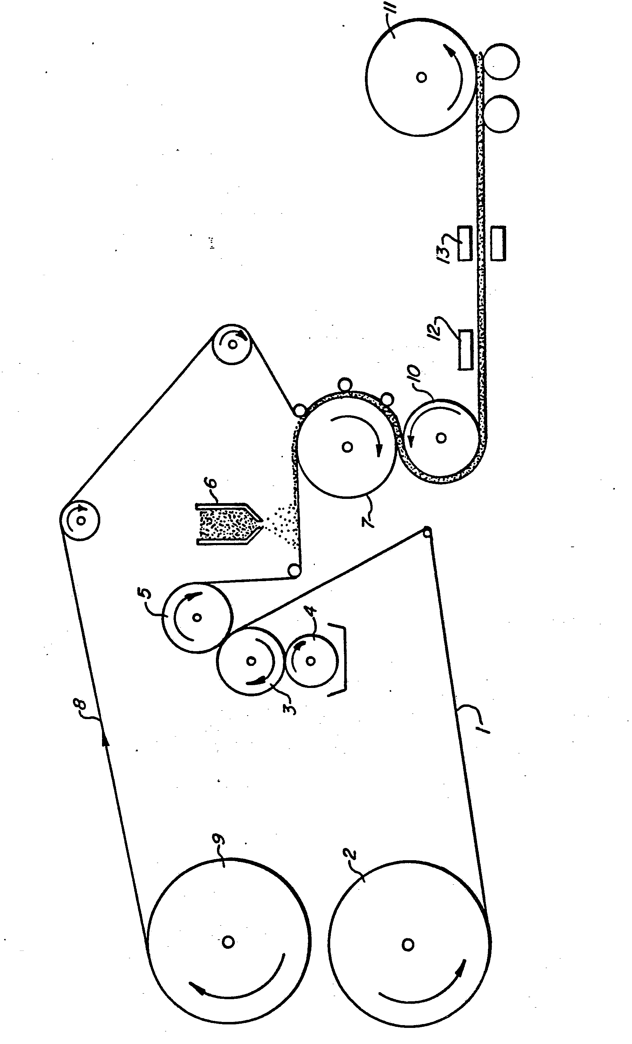

The invention is further described in detail with reference to the drawing which is a schematic flow sheet illustrating a suitable arrangement of apparatus for producing a laminated product in accordance with the invention. Conventional equipment known in the art for producing laminated tissue products can be utilized and variously arranged.

With reference to the drawing, an elongated sheet or web 1 of a suitable substrate tissue is unwound from an unwind stand 2 and passes over a moistening roll 3 of known type, such as a high polished chrome moistening roll. The substrate is guided and maintained in contact with moistening roll 3 by means of conveying or drive roller 5. Water pickup roll 4 serves to continuously moisten roll 3. Drive roll 5 serves to continuously moisten roll 3 at a constant speed relative to the speed of travel of substrate tissue 1 so as to apply a desired amount of moisture thereto. After being sufficiently wetted, the substrate tissue 1 passes under a powder scattering device 6, such as a vibratory feeder, which evenly distributes a layer of an absorbent material on the surface thereof and then over heated metal laminator roll 7. Simultaneously, a second or overlay sheet or web 8 of tissue passes from unwind stand 9 over the laminator roll 7. The webs 1 and 8 are guided into the nip of laminator roll 7 and a hydraulically loaded rubber covered soft roll 10 to provide the heat and pressure necessary to achieve the laminate bond. Generally the laminator roll 7 and the soft roll 10 are set to exert a pressure of from 0.703-1.406 kilogram per square centimeter on the laminate passing through the nip, depending upon the hardness and compressibility of the lamina. The laminate then goes to a surface winder 11 wherein the laminate is formed into a usable roll form. Preferably, but optionally, the laminate can be passed by a moisture measuring device 12 for determining moisture of the laminate and an on-line scanning device 13 for continuous weight measurement. The production line is operated as a unit with sufficient auxiliary devices such as idler rolls, spreader bars, tension and speed control devices as known in the art to function as a high speed production line.

The substrate or backing sheet used to form the laminate is a cellulosic tissue having wet strength properties which allow it to be wetted to a desired moisture content while permitting it to be handled and formed into a laminate on equipment conventionally used for forming tissue laminates. Thus, the substrate tissue employed is an air-laid or wet-laid cellulosic tissue having basis weights from about 32 to 81 grams per square meter and wet strength characteristics of about 15 to 50% of that of the dry tensile strength of the tissue (Tappi T-456-OM-82). One presently preferred substrate or backing sheet material for use in accordance with the invention is an air-laid nonwoven cellulosic tissue produced by James River Corporation under the trade designation "Airtex" SC-130. This tissue has the following characteristics: Basis weight - grams per square meter, 46-51; Caliper-Mils (4 ply), 105-135; Tensile - grams per 7.62 centimeters wide strip CD wet, 550-700; and Water Absorbency Rate - seconds per 1.0 milliliter, 0-4. These tissue substrates exhibit desired characteristics with respect to the necessary wet strength and have a relatively rough or textured surface and are somewhat hydrophobic. The described substrate tissues exhibit sufficiently high wet strengths to permit high line speeds of wetted webs during production. They are somewhat hydrophobic in nature since they retain sufficient water so as to tackify the dry absorbent to provide good laminate bonding while at the same time do not become soggy webs which frequently break during production. Also, these substrate tissues do not retain excessive amounts of water so as to seriously reduce the liquid absorbing capacity of the laminate product.

The overlay tissue lamina need not have the wet strength characteristics of the substrate or backing sheet and can be selected from a variety of cellulosic tissues having basis weights from about 15 to 30 grams per square meter. A particularly preferred overlay tissue is a wet-laid cellulosic material produced by Lincoln Pulp & Paper Company and identified as Code 290. This tissue has a basis weight (grams per square meter) of 17.4.

The absorbents used in the laminated structures are granular or powdered materials which have the ability to absorb liquids, including body fluids. These absorbents are generally hydrophilic polymeric materials which exhibit some adhesive properties when moistened with water. Hydrophilic polymers which exhibit the ability to absorb large quantities of liquids, i.e. in excess of 15 parts of liquid per part thereof, are often referred to in the art as super absorbents and generally fall into three classes, namely, starch graft copolymers, cross-linked carboxymethylcellulose derivatives and modified hydrophilic polyacrylates. It is preferred to employ a super absorbent in the laminated structures of this invention. Many such liquid absorbing materials known to the art can be used, including, for example, such materials as disclosed in United States patents Nos. 3,661,815 (Grain Processing Corporation's

Polymer 35-A-100, now sold under the tradename "Water-Lock"), 3,935,099, 4,076,663, 4,090,013 and 4,293,609.

The absorbent material is generally employed in the laminate ("add-on") according to this invention in the range of about 1 to not above about 8 grams per square foot of substrate tissue. The "add-on" of the absorbent material is preferably within the range of from about 40 to 60% of the total weight of the finished laminate. In order to achieve uniform feeding and deposition with conventional feeding devices used in the tissue laminating art, the absorbent is preferably employed in particle sizes of between -30 mesh and +200 mesh (U.S. standard sieve).

To achieve the objectives of this invention, it has been found that control of the moisture applied to the substrate tissue together with the amount of heat applied to the joined tissue layers is critical with respect to effective bonding of the laminate and distribution of the absorbent therein. Generally, a 20% moisture content by weight based on the amount of absorbent to be employed provides optimum bonding of the laminate at commercial line speeds (60-90 meters per minute) while utilizing a surface temperature of about 150° C. on the heated metal laminator roll (Roll #7 on the drawing). Decreasing the moisture content to 10% or less on the substrate tissue results in insufficient moisture to tackify the polymer surface resulting in poor bonding while moisture add-on contents of 30% and above produce a soggy web which may tend to block or stick together on unwinding the finished roll. The amount of moisture to be added can be easily correlated and determined by monitoring the moisture content of the finished laminate. Generally, a moisture content in the finished laminate of from about 5% to 14% by weight is desired and thus when the final moisture content of the laminate falls below about 5% or above 14%, a greater or lesser amount of moisture is applied to the substrate lamina prior to application of the absorbent material.

The amount of heat transferred to the laminate is primarily a function of line speed and the surface temperature of the heated metal laminator roll. As heat is applied to the backside of the moistened substrate tissue (1), moisture tends to be driven through the applied absorbent toward the cooler top or overlay tissue (8). The pressure achieved in the nip formed by the heated metal laminator roll 7 and the rubber covered pressure roll 10 then sets the laminate bond. It has been observed that the application of too much heat caused by slow operating speeds or high surface temperatures results in a too rapid loss of moisture resulting in a poorly bonded laminate. Conversely, the application of too little heat caused by high machine speeds or low surface temperatures results in the absorbent being bonded only to the lower substrate tissue and little, if any, bond to the top or overlay tissue. The surface temperature of the laminator roll 7 should be in the range of about 120 to 165° C. with the temperatures in the upper portion of the range being employed when the substrate tissue contains higher moisture contents. A particularly preferred combination is to use a surface temperature of about 150° C. in the laminator roll when the moisture content of the substrate tissue is about 20% by weight of absorbent.

The following examples further illustrate the invention and the advantages thereof.

EXAMPLE I

A laminated tissue article was produced employing "Airtex" SC-130 as the substrate tissue. A production line generally similar to that shown in the drawing was utilized and the line was operated at a web speed of approximately 61 meters per minute. The substrate tissue was wetted with water to a moisture content of 20% by weight of absorbent polymer used to produce the laminate. A sodium polyacrylate absorbent polymer ("Water-Lock" J-500 available from Grain Processing Corporation) was distributed on the wetted substrate to provide 42.9-48.4 grams per square meter of laminate. A non-wet strength tissue (Code 290 available from Lincoln Pulp & Paper Company) was applied as the top or overlay tissue and a laminate formed by passing the two tissue layers over a heated laminator roll, the surface temperature of which was maintained at approximately 150° C. The moisture content of the finished laminate was 11-11.5% by weight.

## EXAMPLE II

A laminate was prepared following the procedure of Example I except that "Airtex" SC-130 was used as the tissue for both lamina (substrate and overlay) of the product. The moisture of the finished laminate was approximately 12.5-13%.

## EXAMPLE III

A laminate was employed following the procedure of Example I except that a hydrolyzed starch polyacrylonitrile graft copolymer ("Water-Lock" A-100 available from Grain Processing Corporation) was used as the absorbent.

## EXAMPLE IV

A laminate was prepared following the procedure of Example I except that a wet-laid toweling ("Hi-Loft" available from Scott Paper Company) was used as the wetted substrate tissue.

The laminates produced in the aforementioned examples were characterized by subjective tests for bond and hand while amounts of absorbent applied and absorbency characteristics were determined analytically. The results of these tests are shown in Table I below.

0164743

- 10 -

## TABLE 1

| Laminate Composition | Examples | | | |
|---|---|---|---|---|
| | I | II | III | IV |
| Type of overlay | low wet strength tissue | SC-130 | low wet strength tissue | low wet strength tissue |
| Polymer type | J-500 | J-500 | A-100 | J-500 |
| Polymer Coverage $g/m^2$ | 46.3 | 53.8 | 48.4 | 39.8 |
| Type of substrate | SC-130 | SC-130 | SC-130 | Hi-Loft |
| **Results** | | | | |
| Laminate Bond | Excellent | Good | Good | Fair |
| Hand | Soft | Very Soft & pliable | Soft | Somewhat stiff |
| Absorbency: grams 1% saline absorbed by 51.6 sq. cm. laminate | | | | |
| - in 15 seconds | 15.8 | 19.5 | 6.8 | 14.1 |
| - in 180 seconds | 17.1 | 21.3 | 14.4 | 15.6 |

While the present invention is described with reference to two sheets or lamina, it is apparent that multiple layers of tissue can be used in any combination of tissue-polymer-tissue, tissue-tissue-polymer-tissue-tissue, etc. in single or multiple plies as long as the water can be made to penetrate the tissue and moisten the absorbing agent to render it adhesive. With the present invention laminated products containing an adsorbent can be produced at high speeds and the products exhibit very good bond and desirable attributes as to hand feel. Furthermore, by following the teachings of this invention, difficulties with wrinkling and tearing are avoided as taught in the art if pre-moistened tissue were to be utilized.

Those modifications and equivalents which fall within the spirit of the invention are to be considered a part thereof.

What is claimed is:

1.    A process for producing a liquid absorbing laminated structure which comprises:

(a)    moistening with water a first layer of cellulosic tissue having a basis weight from about 32 to about 81 grams per square meter and a wet strength of about 15 to about 50% of the dry tensile strength,

(b)    distributing in dry form onto said first layer of cellulosic tissue a liquid absorbing material which when moistened with water exhibits adhesive properties,

(c)    superimposing a second layer of cellulosic tissue over said liquid absorbing material, and

(d)    then applying heat and pressure to force together said first and second layers of cellulosic tissue whereby a laminated structure is produced having the water absorbing agent distributed therein in substantially immobilized condition.

2.    A process in accordance with claim 1 wherein in step (a) the first layer of cellulosic tissue is moistened with water to a moisture content of from about 10 to about 30% based on the weight of liquid absorbing material.

3.    A process in accordance with claim 1 wherein in step (a) the first layer of cellulosic material is moistened with water to a moisture content of about 20% based on the weight of liquid absorbing material.

4.    A process in accordance with claim 1 wherein in step (b) the liquid absorbing material is employed in an amount of from about 10.8 to 86 grams per square meter of the said first layer of cellulosic tissue.

5.    A process in accordance with claim 1 wherein in step (b) the liquid absorbing material is employed in an amount of from about 40 to 60% by weight of the finished laminate.

6.    A process in accordance with claim 1 wherein the heat which is applied in step (d) is in the range from about 120 to 165° C.

7.    A process in accordance with claim 1 wherein the moisture content of the laminated structure is controlled to within the range from about 5 to 14%.

0164743